# EUROPEAN PATENT APPLICATION

(11) **EP 3 722 307 A1**
(43) Date of publication of application: **14.10.2020**
(21) Application number: 19168597.3
(22) Date of filing: 11.04.2019
(51) Int. Cl.: C07K 14/435

(54) **TICK VACCINE FOR COMPANION ANIMALS**

(71) Applicant: Beaphar B.V., 8101 BX Raalte (NL)
(72) Inventor: De La Fuente Garcia, Jose de Jesus, 8101 BX Raalte (NL); Contreras Rojo, Marinela, 8101 BX Raalte (NL); Villar Rayo, Margarita Maria, 8101 BX Raalte (NL); Zivkovic Baks, Zorica, 8101 BX Raalte (NL)
(74) Representative: V.O.

(57) **Abstract**

The present invention describes peptides and compositions for control and/or prevention against tick infestations in companion animals, particularly dogs and other susceptible wild and domestic hosts. The present invention further describes nucleic acids, proteins, host cells, vectors and methods of using said vaccine for the control and/or prevention of tick infestations, preferably in companion animals, particularly dogs and other susceptible wild and domestic hosts.

## Description

### FIELD OF THE INVENTION

The present invention broadly relates to the field of immunology. More specifically, the invention relates to a composition to be used in the control and/or prevention of tick infestations in animals. In particular, the vaccine is directed towards peptides identified from larvae and salivary glands of ticks to be used as a vaccine in in companion animals, particularly dogs and other susceptible wild and domestic hosts.

### BACKGROUND

Vector-borne diseases (VBDs) represent a growing burden for human and animal health worldwide. Ticks (Acari: Ixodida) are obligate hematophagous arthropod ectoparasites that are second to mosquitoes as vectors of pathogens causing diseases in humans and the first cause of VBDs in domestic animals. Among the most prevalent tick-borne diseases (TBDs), Lyme disease caused by some species of the *Borrelia burgdorferi* sensu lato (s.1.) complex and anaplasmosis caused by *Anaplasma* spp. constitute a growing burden for humans, companion animals, and farm animals worldwide.

The tick species (Acari: Ixodidae), *Ixodes ricinus* (Linnaeus, 1758) and *Dermacentor reticulatus* (Fabricius, 1794) infest humans, companion animals and other domestic and wild animals, and transmit disease-causing pathogens such as *Borrelia* spp. (Lyme disease and various borreliosis), tick-borne encephalitis virus (TBEV; tick-borne encephalitis), *Anaplasma phagocytophilum* (human and animal anaplasmosis), *Francisella tularensis* (tularemia), *Rickettsia* spp. (human and animal rickettsiosis), Omsk hemorrhagic fever virus (OHFV; Omsk hemorrhagic fever), and *Babesia canis* (canine babesiosis) (de la Fuente, J., et al., 2015. Vet Parasitol. 208: 26-29; Glickman, L.T., et al., 2006. Vector Borne Zoonotic Dis. 6, 14-23; Beugnet and Marié, 2009. Vet Parasitol. 163, 298-305).

Several approaches have been implemented for reducing the risk of TBDs (de la Fuente, J., et al., 2017. Vaccine 35: 5089-5094; de la Fuente, J., 2018. Ticks and Tick-Borne Diseases 9: 1354-1357). These approaches include the use of chemical acaricides, which have been only partially successful and often accompanied by serious drawbacks including the selection of acaricide-resistant ticks and contamination of the environment and animal products with residues, the use of botanical acaricides and repellents, entomopathogenic fungi and the education about recommended practices to reduce exposure to ticks and available options for the management of drug resistance. Additionally, integrated control programs that include habitat management and the genetic selection of hosts with higher resistance to ticks have been also proposed to reduce the use of acaricides for the control of tick infestations. Recent developments have suggested the possibilities of combining chemicals with repellency and parasiticidal activity to reduce the risk of TBDs. This approach intends to prevent both vector infestations and pathogen transmission. However, major difficulties such as long-lasting effect and safety for human and animal use encourage the development of vaccines, which could induce a long-lasting protective immune response against vector infestation and pathogen infection and transmission.

Complete tick eradication by using acaricides does not appear to be a realistic goal for several reasons including (i) the possible elimination of enzootic stability for TBPs, which would result in highly susceptible populations for TBDs, (ii) the difficulty in sustaining it during conditions favorable for tick population expansion, and (iii) the effect on the reduction in biodiversity and the arsenal of bioactive compounds that might be isolated from ticks (de la Fuente, J., et al., 2017. Vaccine 35: 5089-5094; de la Fuente, J., 2018. Ticks and Tick-Borne Diseases 9: 1354-1357). However, the control of tick populations and transmitted pathogens is essential to reduce the burden of TBDs for human and animal health.

Vaccines constitute one of the greatest advances in science with a substantial impact on improving human and animal health. Vaccines for the control of TBDs have posed challenges due to, among other limitations known in the art, the alleged impossibility of preventing tick infestations and consequently the possibility of pathogen transmission. For those involved in the development of vaccines for the control of tick infestations and TBDs, these limitations constitute a challenge that has been approached by developing new platforms for the identification and characterization of candidate tick-derived and pathogen-derived protective peptides (de la Fuente, J., et al., 2017. Vaccine 35: 5089-5094; de la Fuente, J., 2018. Ticks and Tick-Borne Diseases 9: 1354-1357).

For at least the above reasons, the development of vaccines against tick infestations and conditions associated with tick infestations is highly desirable. Vaccines could be developed to target different tick developmental stages and functions on various hosts with the advantage of avoiding environmental contamination and selection of pesticide resistant arthropod vectors while improving animal welfare and production. Without wishing to be bound by theory, the hypothesis behind tick vaccine protective capacity is that ticks feeding on immunized hosts ingest antibodies specific for the target peptide that could reduce the levels and biological activity of the target peptide and/or lead to interactions with conserved epitopes in other proteins resulting in reduced tick feeding, development and reproduction.

The first vaccines for the control of cattle tick infestations became commercially available in the early 1990s (de la Fuente J., et al., 2007. Anim Health Res Rev 8: 23-28). These vaccines contained the *Rhipicephalus microplus* BM86 or BM95 recombinant peptides and their use demonstrated that vaccines could constitute an effective component of the integrated programs for the control of TBDs. These vaccines were not designed to prevent tick infestations, but to reduce tick populations and the prevalence of tick-borne pathogens (TBPs) by affecting feeding, reproduction and development of ticks feeding on immunized animals and ingesting with the blood meal peptide-specific antibodies that interact with and affect protein function. However, this vaccine is effective only against cattle ticks. The search for new vaccine candidates has been going on for many years. Identification of novel, protective peptides is the limiting step in increasing the effectiveness of any potential vaccine to be used to prevent tick infestations. As stated, ticks feed on blood of the host and therefore may manipulate the host immune system to complete feeding. Due to poor knowledge of the mechanisms and pathology of tick infestations, target identification for prevention and treatment of this infection have not been successful for most tick species including those affecting domestic and wild animals.

Therefore, at least one of the objectives of the present invention is to identify suitable peptides for a novel vaccine, and to provide compositions comprising said peptides as a novel vaccine that is capable of inducing a protective immune response, especially in companion animals (particularly dogs and cats), and other susceptible wild and domestic hosts. In addition, it is desired that this vaccine provides a degree of protection against tick infestations, in particular to improve animal health and welfare. Moreover, as ticks are vectors of multiple pathogenic agents, the reduction on the incidence of tick infestations afforded by the vaccines may concurrently reduce the incidence of tick-borne diseases in susceptible animals.

### SUMMARY OF THE INVENTION

In one aspect, the invention pertains to an isolated peptide identified in the tick species *Ixodes ricinus* and/or *Dermacentor reticulatus,* wherein said peptide is identified in both a larva of said tick species, and a salivary gland of an adult tick of the same tick species as said larva, wherein said peptide comprises an amino acid subsequence of SEQ ID 1, wherein said peptide consists of a number of amino acid residues in a range of from 10 to 350, preferably in a range of from 20 to 300.

In another aspect, the invention relates to a method for identifying a protective peptide, comprising the steps of:
a) providing at least one tick larva, and a salivary gland of an adult tick, wherein the tick is *Ixodes ricinus* and/or *Dermacentor reticulatus*;
b) identifying at least one peptide that is expressed in both the tick larva, and the salivary gland of the adult tick.

In another aspect, the invention relates to an isolated peptide obtainable by the method for identifying a protective peptide as defined herein.

In another aspect, the invention pertains to a composition comprising an immunologically effective amount of at least one peptide according to the invention.

In another aspect, the invention relates to a peptide according to the invention or a composition according to the invention, for use as a medicament.

In yet another aspect, the invention relates to a peptide according to the invention or the composition according to the invention, for use in the control or prophylactic treatment of a tick infestation and/or a condition associated with a tick infestation, in a subject.

In yet a further aspect, the invention pertains to a vector comprising a nucleic acid sequence encoding a peptide according to the invention.

In a further aspect still, the invention relates to an *in vitro* host cell comprising a vector according to the invention.

In yet a further aspect, the invention pertains to a use of a peptide according to the invention, or a composition according to the invention, in the preparation of a vaccine.

In yet another aspect, the invention relates to a method of controlling or the prophylactic treatment of a tick infestation and/or a condition associated with a tick infestation, in a subject, wherein the controlling or the prophylactic treatment is as defined herein in accordance with the invention, and wherein the subject is as defined herein in accordance with the invention.

### BRIEF DESCRIPTION OF THE FIGURES

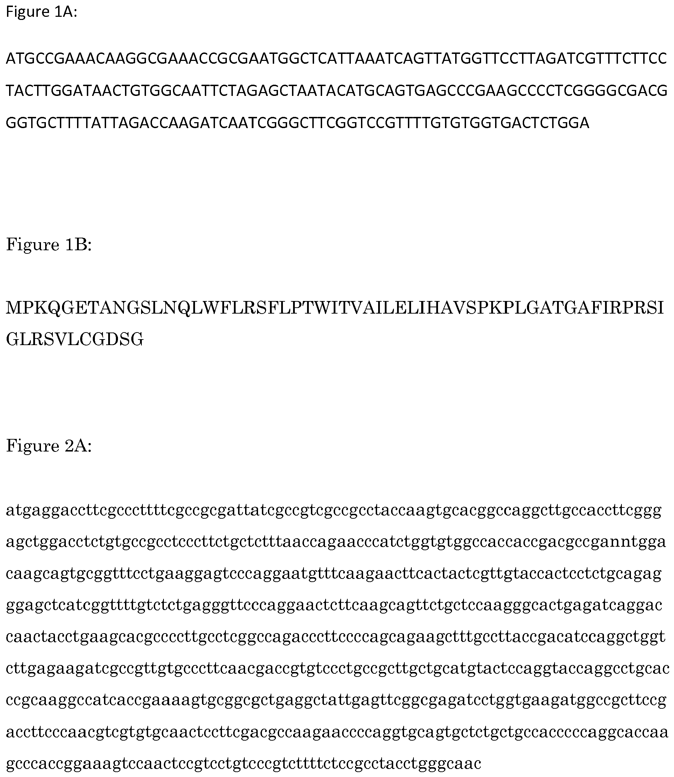

Figure 1A: Nucleotide coding sequence of the identified tick peptide SEQ ID 082.
Figure 1B: Amino acid sequence of the identified tick peptide SEQ ID 082.
Figure 2A: Nucleotide coding sequence of the identified tick peptide SEQ ID 216.

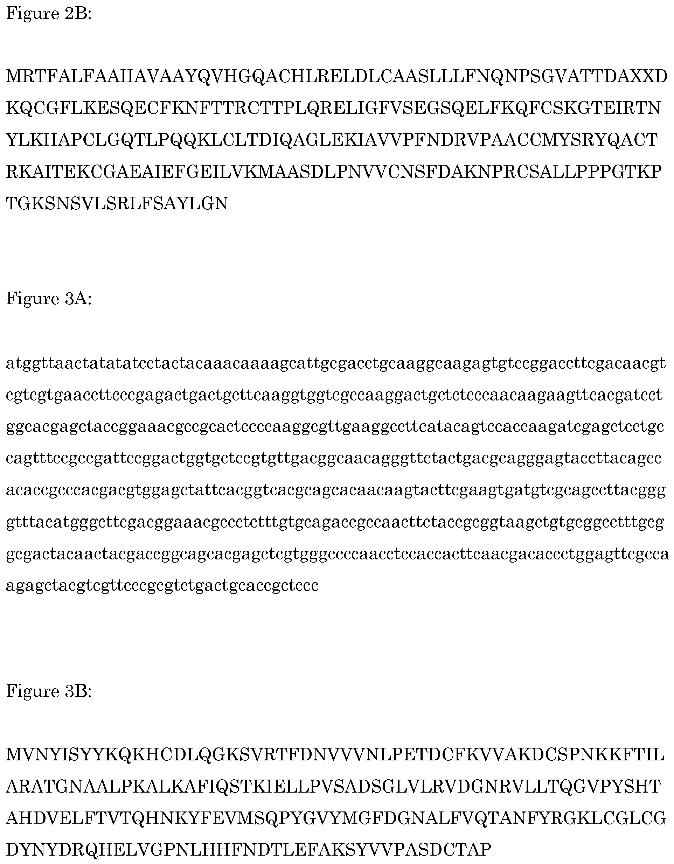

Figure 2B: Amino acid sequence of the identified tick peptide SEQ ID 216.
Figure 3A: Nucleotide coding sequence of the identified tick peptide SEQ ID 391.
Fiqure 3B: Amino acid sequence of the identified tick peptide SEQ ID 391.

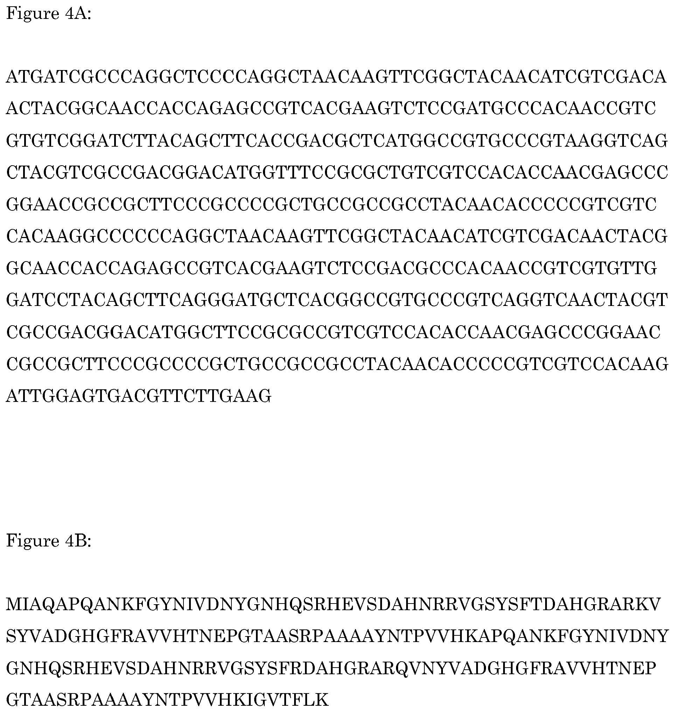

Figure 4A: Nucleotide coding sequence of the identified tick peptide SEQ ID 749.
Figure 4B: Amino acid sequence of the identified tick peptide SEQ ID 749.

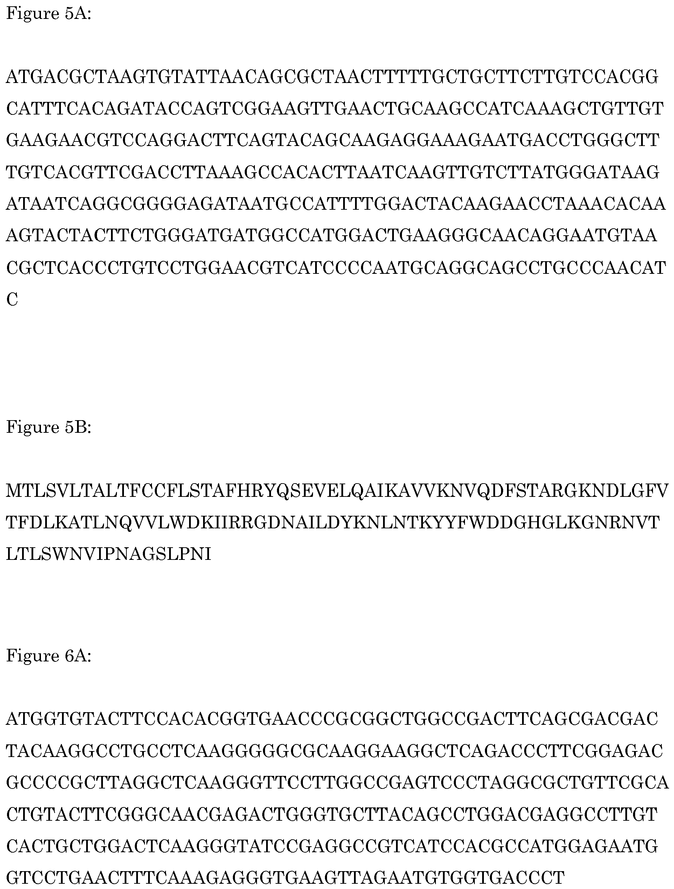

Figure 5A: Nucleotide coding sequence of the identified tick peptide SEQ ID S2.
Figure 5B: Amino acid sequence of the identified tick peptide SEQ ID S2.
Figure 6A: Nucleotide coding sequence of the identified tick peptide SEQ ID S8.

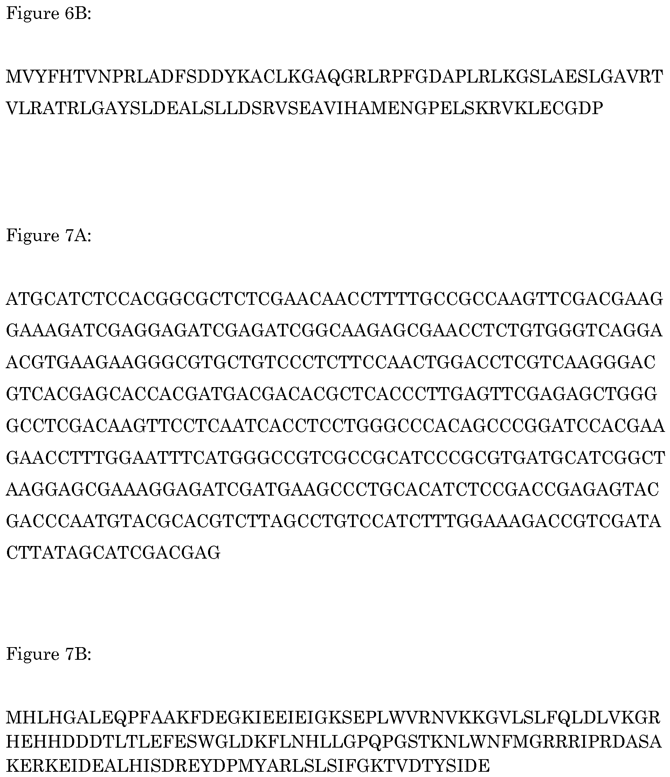

Figure 6B: Amino acid sequence of the identified tick peptide SEQ ID S8.
Figure 7A: Nucleotide coding sequence of the identified tick peptide SEQ ID S10.
Figure 7B: Amino acid sequence of the identified tick peptide SEQ ID S10.

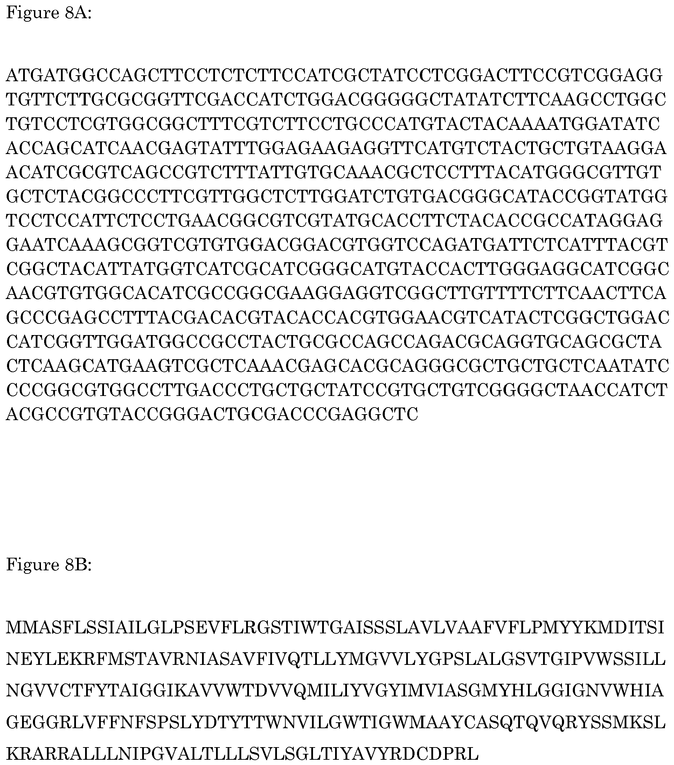

Figure 8A: Nucleotide coding sequence of the identified tick peptide SEQ ID S12.
Figure 8B: Amino acid sequence of the identified tick peptide SEQ ID S12.

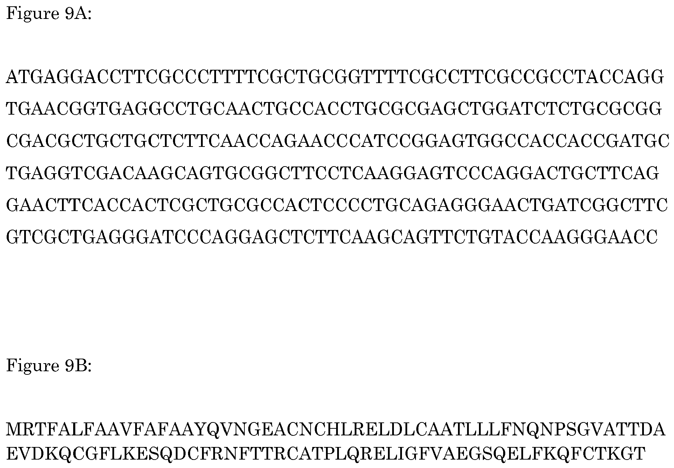

Figure 9A: Nucleotide coding sequence of the identified tick peptide SEQ ID S14.
Figure 9B: Amino acid sequence of the identified tick peptide SEQ ID S14.

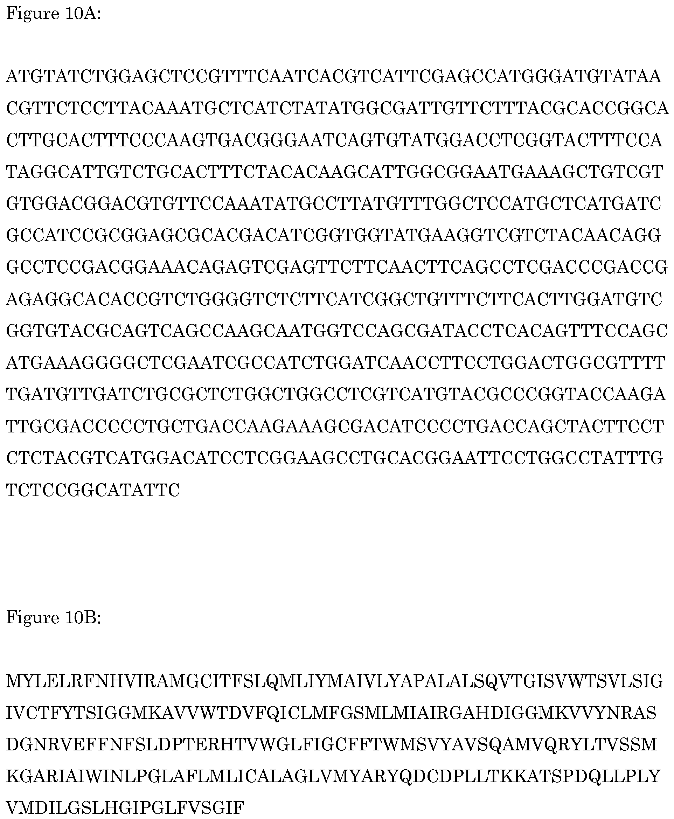

Figure 10A: Nucleotide coding sequence of the identified tick peptide SEQ ID S16.
Figure 10B: Amino acid sequence of the identified tick peptide SEQ ID S16.

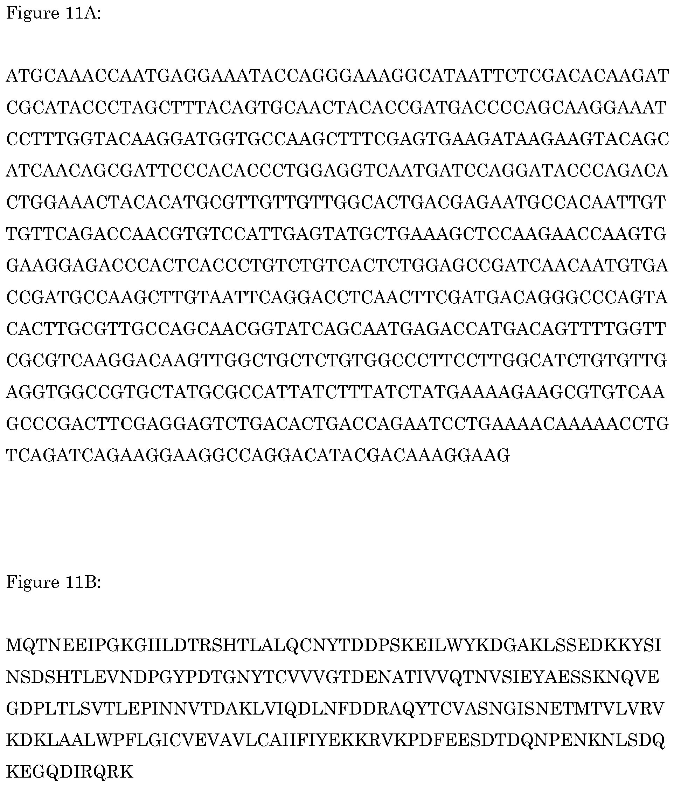

Figure 11A: Nucleotide coding sequence of the identified tick peptide SEQ ID S17.
Figure 11B: Amino acid sequence of the identified tick peptide SEQ ID S17.

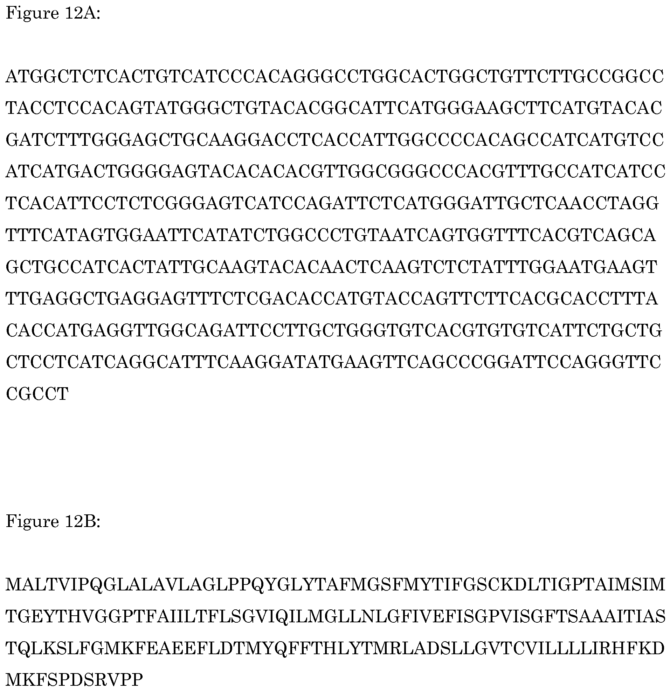

Figure 12A: Nucleotide coding sequence of the identified tick peptide SEQ ID S18.
Figure 12B: Amino acid sequence of the identified tick peptide SEQ ID S18.

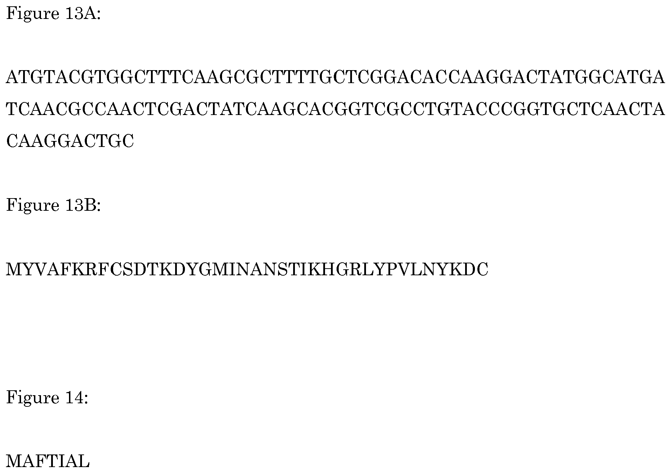

Figure 13A: Nucleotide coding sequence of the identified tick peptide SEQ ID S20.
Figure 13B: Amino acid sequence of the identified tick peptide SEQ ID S20.
Figure 14: Amino acid sequence of the identified tick peptide SEQ ID 1.
Figure 15: Example of recombinant tick peptides produced in *E. coli.* 12% SDS-PAGE stained with Coomassie blue.
Figure 16. Graphical representation of the results of vaccination with tick peptides in reducing *I. ricinus* and *D. reticulatus* infestations in dogs.
Figure 17A. Immunogenicity of tick protective peptides in vaccinated rabbits.
Figure 17B. Immunogenicity of tick protective peptides in vaccinated dogs.

### DETAILED DESCRIPTION OF THE INVENTION

The invention is based on the judicious insight to provide a peptide as defined herein in relation to the invention that can be used to prepare a vaccine that achieves at least one of the abovementioned objectives and/or desires. Said peptide is an isolated peptide identified in the tick species *Ixodes ricinus* and/or *Dermacentor reticulatus,* wherein said peptide is identified in both a larva of said tick species, and a salivary gland of an adult tick of the same tick species as said larva, wherein said peptide comprises an amino acid subsequence of SEQ ID 1, wherein said peptide consists of a number of amino acid residues in a range of from 10 to 350, preferably in a range of from 20 to 300.

Without wishing to be bound by theory, the inventors believe that *I. ricinus* and *D. reticulatus* acquire infection when larvae feed on infected hosts and after transtadial transmission, pathogen are secreted from salivary glands during feeding of adult ticks. The inventors believe that affecting these two developmental stages reduces both tick infestations and pathogen transmission. Surprisingly, it was found that, in one aspect, peptides according to the invention are able to achieve this, in particular when used as a vaccine.

It will be understood that when a peptide according to the invention is identified in the tick species *Ixodes ricinus* or *Dermacentor reticulatus,* wherein said peptide is identified in both a larva of said tick species, and a salivary gland of an adult tick of the same tick species as said larva, then the peptide is identified in either a tick larva and an adult tick from the species *Ixodes ricinus,* or in a tick larva and an adult tick from the species *Dermacentor reticulatus.*

It will be understood that when a peptide according to the invention is identified in the tick species *Ixodes ricinus* and *Dermacentor reticulatus,* wherein said peptide is identified in both a larva of said tick species, and a salivary gland of an adult tick of the same tick species as said larva, then the peptide is identified in both a tick larva and an adult tick from the species *Ixodes ricinus,* and in both a tick larva and an adult tick from the species *Dermacentor reticulatus.*

The skilled person is aware that a tick undergoes several life stages. Typically, a tick goes through four life stages: egg, larva, nymph, and adult. The skilled person is able to distinguish ticks from every life stage.

The term "amino acid sequence" as used herein refers to a sequence of amino acid residues. Likewise, the term "amino acid subsequence" as used herein refers to a subsequence of amino acid residues. A subsequence is typically defined as "a *sequence that can be derived from another sequence by deleting some or no elements without changing the order of the remaining elements."* (source: Wikipedia; https://en.wikipedia.org/wiki/Subsequence). For example, "OS" is a subsequence of "HORSE", by deleting the H, R, and E. By contrast, "ROS" is not a subsequence of "HORSE", as it requires changing the order in which R and O appear.

Also in relation to the amino acid subsequence of the invention, it will be understood that commonly, amino acid sequences are considered to start at the N-terminus. In other words, in an amino acid sequence of "MCGGS", using the one-letter reference code for amino acid residues, the methionine residue is at the N-terminus and the serine residue at the C-terminus.

In a preferred embodiment, the peptide according to the invention has at least about 80% sequence identity with an amino acid sequence that is selected from the group consisting of SEQ ID 082, SEQ ID 216, SEQ ID 391, SEQ ID 749, SEQ ID S2, SEQ ID S8, SEQ ID S10, SEQ ID S12, SEQ ID S14, SEQ ID S16, SEQ ID S17, SEQ ID S18, SEQ ID S20, and variants or immunogenic fragments thereof.

In a preferred embodiment, the peptide according to the invention has at least about 85% sequence identity with an amino acid sequence that is selected from the group consisting of SEQ ID 082, SEQ ID 216, SEQ ID 391, SEQ ID 749, SEQ ID S2, SEQ ID S8, SEQ ID S10, SEQ ID S12, SEQ ID S14, SEQ ID S16, SEQ ID S17, SEQ ID S18, SEQ ID S20, and variants or immunogenic fragments thereof.

In a preferred embodiment of the present invention the peptide according to the invention has at least about 90% sequence identity with an amino acid sequence that is selected from the group consisting of SEQ ID 082, SEQ ID 216, SEQ ID 391, SEQ ID 749, SEQ ID S2, SEQ ID S8, SEQ ID S10, SEQ ID S12, SEQ ID S14, SEQ ID S16, SEQ ID S17, SEQ ID S18, SEQ ID S20, and variants or immunogenic fragments thereof.

In a preferred embodiment of the present invention the peptide according to the invention has at least about 95% sequence identity with an amino acid sequence that is selected from the group consisting of SEQ ID 082, SEQ ID 216, SEQ ID 391, SEQ ID 749, SEQ ID S2, SEQ ID S8, SEQ ID S10, SEQ ID S12, SEQ ID S14, SEQ ID S16, SEQ ID S17, SEQ ID S18, SEQ ID S20, and variants or immunogenic fragments thereof.

In a preferred embodiment, the peptide according to the invention has a sequence identity of at least about 80%, 85%, 90% or 95% as compared to SEQ ID 082 or a variant or immunogenic fragment thereof. In a preferred embodiment, the peptide according to the invention has a sequence identity of SEQ ID 082.

In a preferred embodiment, the peptide according to the invention has a sequence identity of at least about 80%, 85%, 90% or 95% as compared to SEQ ID 216 or a variant or immunogenic fragment thereof. In a preferred embodiment, the peptide according to the invention has a sequence identity of SEQ ID 216.

In a preferred embodiment, the peptide according to the invention has a sequence identity of at least about 80%, 85%, 90% or 95% as compared to SEQ ID 391 or a variant or immunogenic fragment thereof. In a preferred embodiment, the peptide according to the invention has a sequence identity of SEQ ID 391.

In a preferred embodiment, the peptide according to the invention has a sequence identity of at least about 80%, 85%, 90% or 95% as compared to SEQ ID 749 or a variant or immunogenic fragment thereof. In a preferred embodiment, the peptide according to the invention has a sequence identity of SEQ ID 749.

In a preferred embodiment, the peptide according to the invention has a sequence identity of at least about 80%, 85%, 90% or 95% as compared to SEQ ID S2 or a variant or immunogenic fragment thereof. In a preferred embodiment, the peptide according to the invention has a sequence identity of SEQ ID S2.

In a preferred embodiment, the peptide according to the invention has a sequence identity of at least about 80%, 85%, 90% or 95% as compared to SEQ ID S8 or a variant or immunogenic fragment thereof. In a preferred embodiment, the peptide according to the invention has a sequence identity of SEQ ID S8.

In a preferred embodiment, the peptide of the present invention comprises a peptide having a sequence identity of at least about 80%, 85%, 90% or 95% as compared to SEQ ID S10 or a variant or immunogenic fragment thereof. In a preferred embodiment, the peptide of the present invention comprises a peptide having a sequence identity of SEQ ID S10.

In a preferred embodiment, the peptide according to the invention has a sequence identity of at least about 80%, 85%, 90% or 95% as compared to SEQ ID S12 or a variant or immunogenic fragment thereof. In a preferred embodiment, the peptide according to the invention has a sequence identity of SEQ ID S12.

In a preferred embodiment, the peptide according to the invention has a sequence identity of at least about 80%, 85%, 90% or 95% as compared to SEQ ID S14 or a variant or immunogenic fragment thereof. In a preferred embodiment, the peptide according to the invention has a sequence identity of SEQ ID S14.

In a preferred embodiment, the peptide according to the invention has a sequence identity of at least about 80%, 85%, 90% or 95% as compared to SEQ ID S16 or a variant or immunogenic fragment thereof. In a preferred embodiment, the peptide according to the invention has a sequence identity of SEQ ID S16.

In a preferred embodiment, the peptide according to the invention has a sequence identity of at least about 80%, 85%, 90% or 95% as compared to SEQ ID S17 or a variant or immunogenic fragment thereof. In a preferred embodiment, the peptide according to the invention has a sequence identity of SEQ ID S17.

In a preferred embodiment, the peptide according to the invention has a sequence identity of at least about 80%, 85%, 90% or 95% as compared to SEQ ID S18 or a variant or immunogenic fragment thereof. In a preferred embodiment, the peptide according to the invention has a sequence identity of SEQ ID S18.

In a preferred embodiment, the peptide according to the invention has a sequence identity of at least about 80%, 85%, 90% or 95% as compared to SEQ ID S20 or a variant or immunogenic fragment thereof. In a preferred embodiment, the peptide according to the invention has a sequence identity of SEQ ID S20.

In a preferred embodiment, the peptide according to the invention is a recombinant peptide.

In a preferred embodiment, the peptide according to the invention is a secreted peptide. It will be understood that "secreted" does not mean that the specific peptide *per se* has been secreted by a tick. For example, a secreted peptide can be a recombinant peptide, that has been isolated from a bacterial source using standard molecular biology techniques. Rather, "secreted" refers to the normal state of the peptide in a tick, i.e. that if the peptide would have been expressed in a tick, it would normally have been secreted.

In another aspect, the invention relates to a method for identifying a protective peptide, comprising the steps of:
a) providing at least one tick larva, and a salivary gland of an adult tick, wherein the tick is *Ixodes ricinus* and/or *Dermacentor reticulatus*;
b) identifying at least one peptide that is expressed in both the tick larva, and the salivary gland of the adult tick.

In a preferred embodiment, the method for identifying a protective peptide, comprises the steps of:
a) providing at least one tick larva, and a salivary gland of an adult tick, wherein the tick is *Ixodes ricinus* and/or *Dermacentor reticulatus*;
b) identifying at least one peptide that is expressed in both the tick larva, and the salivary gland of the adult tick;
c) isolating the at least one peptide identified in step b).

In a preferred embodiment, the method for identifying a protective peptide comprises the steps of:
a) providing at least one tick larva, and a salivary gland of an adult tick, wherein the tick is *Ixodes ricinus* and/or *Dermacentor reticulatus*;
b) isolating nucleic acid, preferably RNA, from both the tick larva, and the salivary gland of the adult tick;
c) identifying which peptides are encoded by the nucleic acid isolated in step b);
d) identifying at least one peptide that is expressed in both the tick larva, and the salivary gland of the adult tick.

In a preferred embodiment, the method for identifying a protective peptide comprises the steps of:
a) providing at least one tick larva, and a salivary gland of an adult tick, wherein the tick is *Ixodes ricinus* and/or *Dermacentor reticulatus*;
b) isolating nucleic acid, preferably RNA, from both the tick larva, and the salivary gland of the adult tick;
c) identifying which peptides are encoded by the nucleic acid isolated in step b);
d) identifying at least one peptide that is expressed in both the tick larva, and the salivary gland of the adult tick;
e) isolating the at least one peptide identified in step d).

In a preferred embodiment, isolating the at least one peptide in a method for identifying a protective peptide as disclosed herein is performed by isolating the at least one peptide from a tick larva, preferably as defined herein, and/or the salivary gland of an adult tick, preferably as defined herein, and/or by using recombinant techniques. Said recombinant techniques may include, but are not limited to, providing a nucleic acid, preferably a plasmid, encoding for said peptide, transforming a cell, preferably a bacterium, with said nucleic acid, expressing said protein in said cell, and isolating said protein.

In another aspect, the invention pertains to a peptide obtainable by the method for identifying a protective peptide, as disclosed herein. Preferably, said peptide is further as defined herein. For the peptide obtainable by the method for identifying a protective peptide as defined herein, any of the methods for identifying a protective peptide as defined herein are suitable.

In a preferred embodiment, the peptide is obtainable by a method for identifying a protective peptide, comprises the steps of:
a) providing at least one tick larva, and a salivary gland of an adult tick, wherein the tick is *Ixodes ricinus* and/or *Dermacentor reticulatus*;
b) identifying at least one peptide that is expressed in both the tick larva, and the salivary gland of the adult tick;
c) isolating the at least one peptide identified in step b).

In a preferred embodiment, the peptide is obtainable by a method for identifying a protective peptide comprises the steps of:
a) providing at least one tick larva, and a salivary gland of an adult tick, wherein the tick is *Ixodes ricinus* and/or *Dermacentor reticulatus*;
b) isolating nucleic acid, preferably RNA, from both the tick larva, and the salivary gland of the adult tick;
c) identifying which peptides are encoded by the nucleic acid isolated in step b);
d) identifying at least one peptide that is expressed in both the tick larva, and the salivary gland of the adult tick;
e) isolating the at least one peptide identified in step d).

In another aspect, the invention pertains to a composition comprising an immunologically effective amount of at least one peptide according to the invention.

In a preferred embodiment, the composition of the invention further comprises at least one or more additional peptide(s), as described herein in relation to the invention. The term "one or more additional peptide", as used herein, refers to the composition of the invention further comprising one or more different peptides according to the invention, used as part of the composition in addition to the first peptide. I.e., the first peptide is selected from the group consisting of SEQ ID 082, SEQ ID 216, SEQ ID 391, SEQ ID 749, SEQ ID S2, SEQ ID S8, SEQ ID S10, SEQ ID S12, SEQ ID S14, SEQ ID S16, SEQ ID S17, SEQ ID S18, SEQ ID S20, and variants or immunogenic fragments thereof. The second peptide is a different peptide than the first peptide, but is also selected from the group consisting of SEQ ID 082, SEQ ID 216, SEQ ID 391, SEQ ID 749, SEQ ID S2, SEQ ID S8, SEQ ID S10, SEQ ID S12, SEQ ID S14, SEQ ID S16, SEQ ID S17, SEQ ID S18, SEQ ID S20, and variants or immunogenic fragments thereof.

In a preferred embodiment, the one or more additional peptide is identified from tick larvae and/or tick salivary glands.

In a preferred embodiment the one or more additional peptide is a recombinant peptide, and is preferably isolated as described herein.

In a preferred embodiment, the composition according to the invention comprises an immunologically effective amount of at least two different peptides according to the invention. More preferably, the composition according to the invention comprises an immunologically effective amount of at least three different peptides according to the invention. Even more preferably, the composition according to the invention comprises an immunologically effective amount of at least four different peptides according to the invention. Even more preferably still, the composition according to the invention comprises an immunologically effective amount of at least five different peptides according to the invention. Most preferably, the composition according to the invention comprises an immunologically effective amount of at least six different peptides according to the invention.

In a preferred embodiment the composition of the invention further comprises one or more peptides having at least about 80% sequence identity with an amino acid sequence that is selected from the group consisting of SEQ ID 082, SEQ ID 216, SEQ ID 391, SEQ ID 749, SEQ ID S2, SEQ ID S8, SEQ ID S10, SEQ ID S12, SEQ ID S14, SEQ ID S16, SEQ ID S17, SEQ ID S18, SEQ ID S20 and variants or immunogenic fragments thereof.

In a preferred embodiment of the present invention the composition further comprises one or more peptides having at least about 85% sequence identity with an amino acid sequence that is selected from the group consisting of SEQ ID 082, SEQ ID 216, SEQ ID 391, SEQ ID 749, SEQ ID S2, SEQ ID S8, SEQ ID S10, SEQ ID S12, SEQ ID S14, SEQ ID S16, SEQ ID S17, SEQ ID S18, SEQ ID S20, and variants or immunogenic fragments thereof.

In a preferred embodiment of the present invention the composition further comprises one or more peptides having at least about 90% sequence identity with an amino acid sequence that is selected from the group consisting of SEQ ID 082, SEQ ID 216, SEQ ID 391, SEQ ID 749, SEQ ID S2, SEQ ID S8, SEQ ID S10, SEQ ID S12, SEQ ID S14, SEQ ID S16, SEQ ID S17, SEQ ID S18, SEQ ID S20, and variants or immunogenic fragments thereof.

In a preferred embodiment of the present invention the composition further comprises one or more peptides having at least about 95% sequence identity with an amino acid sequence that is selected from the group consisting of SEQ ID 082, SEQ ID 216, SEQ ID 391, SEQ ID 749, SEQ ID S2, SEQ ID S8, SEQ ID S10, SEQ ID S12, SEQ ID S14, SEQ ID S16, SEQ ID S17, SEQ ID S18, SEQ ID S20, and variants or immunogenic fragments thereof.

In a preferred embodiment, the one or more peptide used in the composition of the present invention further comprises a peptide having a sequence identity of at least about 80%, 85%, 90% or 95% as compared to SEQ ID 082 or a variant or immunogenic fragment thereof. In a preferred embodiment, the one or more peptide used in the composition of the present invention further comprises a peptide having a sequence identity of at least about 80%, 85%, 90% or 95% as compared to SEQ ID 216 or a variant or immunogenic fragment thereof. In a preferred embodiment, the one or more peptide used in the composition of the present invention further comprises a peptide having a sequence identity of at least about 80%, 85%, 90% or 95% as compared to SEQ ID 391 or a variant or immunogenic fragment thereof. In a preferred embodiment, the one or more peptide used in the composition of the present invention further comprises a peptide having a sequence identity of at least about 80%, 85%, 90% or 95% as compared to SEQ ID 749 or a variant or immunogenic fragment thereof. In a preferred embodiment, the one or more peptide used in the composition of the present invention further comprises a peptide having a sequence identity of at least about 80%, 85%, 90% or 95% as compared to SEQ ID S2 or a variant or immunogenic fragment thereof. In a preferred embodiment, the one or more peptide used in the composition of the present invention further comprises a peptide having a sequence identity of at least about 80%, 85%, 90% or 95% as compared to SEQ ID S8 or a variant or immunogenic fragment thereof. In a preferred embodiment, the one or more peptide used in the composition of the present invention further comprises a peptide having a sequence identity of at least about 80%, 85%, 90% or 95% as compared to SEQ ID S10 or a variant or immunogenic fragment thereof. In a preferred embodiment, the one or more peptide used in the composition of the present invention further comprises a peptide having a sequence identity of at least about 80%, 85%, 90% or 95% as compared to SEQ ID S12 or a variant or immunogenic fragment thereof. In a preferred embodiment, the one or more peptide used in the composition of the present invention further comprises a peptide having a sequence identity of at least about 80%, 85%, 90% or 95% as compared to SEQ ID S14 or a variant or immunogenic fragment thereof. In a preferred embodiment, the one or more peptide used in the composition of the present invention further comprises a peptide having a sequence identity of at least about 80%, 85%, 90% or 95% as compared to SEQ ID S16 or a variant or immunogenic fragment thereof. In a preferred embodiment, the one or more peptide used in the composition of the present invention further comprises a peptide having a sequence identity of at least about 80%, 85%, 90% or 95% as compared to SEQ ID S17 or a variant or immunogenic fragment thereof. In a preferred embodiment, the one or more peptide used in the composition of the present invention further comprises a peptide having a sequence identity of at least about 80%, 85%, 90% or 95% as compared to SEQ ID S18 or a variant or immunogenic fragment thereof. In a preferred embodiment, the one or more peptide used in the composition of the present invention further comprises a peptide having a sequence identity of at least about 80%, 85%, 90% or 95% as compared to SEQ ID S20 or a variant or immunogenic fragment thereof.

In general, a composition according to the invention is a vaccine that may protect an animal from disease or possible death. It optionally includes one or more additional components that enhance the immunological activity of the active component. The vaccine of the invention could be considered by one of skill in the art as a subunit or peptide vaccine. A vaccine may additionally comprise further components typical to vaccines or compositions, including, for example, an adjuvant or an immunomodulator. The immunogenically active components of a vaccine may comprise subunit vaccines comprising one or more immunogenic components of an peptide, or genetically engineered, mutated or cloned vaccines prepared by methods known to those skilled in the art or further comprise complete live organisms in either their original form, or as attenuated organisms in a modified live vaccine, or organisms inactivated by appropriate methods in a killed or inactivated vaccine. A vaccine may comprise one or more than one of the elements described above.

The composition of the invention may further comprise a suitable pharmaceutical carrier. The term "pharmaceutically acceptable carrier" is intended to include any and all solvents, dispersion media, coatings, antibacterial and antifungal agents, isotonic and absorption-delaying agents, to hosts. The term "carrier" refers to a diluent, adjuvant, excipient, or vehicle with which the pharmaceutical composition is administered. Such pharmaceutical carriers can be sterile liquids, such as water and oils, including those of petroleum, animal, vegetable or synthetic origin, such as peanut oil, soybean oil, mineral oil, Sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained release formulations and the like. The composition can be formulated with traditional binders and carriers such as triglycerides depending on the method of administration. Particular formulations can include standard carriers such as pharmaceutical grades of mannitol, lactose, starch, magnesium stearate, sodium saccharine, cellulose, magnesium carbonate, etc. The formulation should suit the mode of administration. The appropriate carrier is evident to those skilled in the art and will depend in large part upon the route of administration. Additional components that may be present in this invention are adjuvants, preservatives, surface active agents, chemical stabilizers, suspending or dispersing agents. Typically, stabilizers, adjuvants and preservatives are optimized to determine the best formulation for efficacy in the target subject. In a currently preferred embodiment the vaccine is formulated as an emulsion of water in oil.

Preferably, the composition according to the invention also comprises a "vehicle". A vehicle is a device to which the peptide adheres, without being covalently bound to it. Such vehicles are biodegradable nano/micro-particles or -capsules of PLGA(poly-lactide-co-glycolic acid), alginate or chitosan, liposomes, niosomes, micelles, multiple emulsions and macrosols, all known in the art.

In a preferred embodiment the composition of the present invention further comprises a pharmaceutically acceptable adjuvant. There has been an increasing demand for subunit vaccine comprising at least one peptide, such as the vaccine of the present invention. Subunit vaccines can be well tolerated. In addition, subunit vaccines may benefit from the addition of an adjuvant to achieve protective immune responses. The term vaccine adjuvant has been used to define several compounds that enhance the immunogenicity of a co-administered peptide in vivo. As a consequence of this definition, the vaccine adjuvant group is composed of diverse classes of molecules such as microbial products, emulsions, mineral salts, small molecules, microparticles and liposomes that have different mechanisms of action. Examples of adjuvants used in animal vaccines include muramyldipeptides, lipopolysaccharides, several glucans and glycans, animal oil, vegetable oil, mineral oil, Montanide® and Carbopol®.

Without wishing to be bound by theory, it is believed that in general, adjuvants boost a vaccine response by increasing the persistency of the peptide in vivo and/or by targeting innate immune pathways normally associated with response to infection.

In a preferred embodiment the adjuvants in relation to the invention are selected from the group consisting of lipopolysaccharides, glucans, glycans, inactivated protein extracts, oil-in-water emulsion, Freund's adjuvant and acrylic acid polymers. In another preferred embodiment, the pharmaceutically acceptable adjuvant in relation to the invention is selected from the group consisting of muramyl dipeptides, lipopolysaccharides, glucans, glycans, an oil-in-water emulsion, a water-in-oil emulsion, Freund's adjuvant, and acrylic acid polymers. In a preferred embodiment the adjuvant in relation to the invention is a water-in-oil emulsion. In a preferred embodiment the oil in the water-in-oil-emulsion is selected from an animal, vegetable or mineral oil.

In another aspect of the invention, the peptide or composition of the present invention is for use as a medicament.

In another preferred embodiment, the peptide or composition of the present invention is for use in the control or prophylactic treatment of a tick infestation and/or a condition associated with a tick infestation, in a subject.

In another preferred embodiment, the peptide or composition of the present invention is for use in the control of a tick infestation and/or a condition associated with a tick infestation, in a subject.

In another preferred embodiment, the peptide or composition of the present invention is for use in the prophylactic treatment of a tick infestation and/or a condition associated with a tick infestation, in a subject.

In another preferred embodiment, the peptide or composition of the present invention is for use in the control or prophylactic treatment of a tick infestation, in a subject.

In another preferred embodiment, the peptide or composition of the present invention is for use in the control of a tick infestation, in a subject.

In another preferred embodiment, the peptide or composition of the present invention is for use in the prophylactic treatment of a tick infestation, in a subject.

In another preferred embodiment, the peptide or composition of the present invention is for use in the control or prophylactic treatment of a condition associated with a tick infestation, in a subject.

In another preferred embodiment, the peptide or composition of the present invention is for use in the control of a condition associated with a tick infestation, in a subject.

In another preferred embodiment, the peptide or composition of the present invention is for use in the prophylactic treatment of a condition associated with a tick infestation, in a subject.

Preferably, the control or prophylactic treatment in relation to the invention comprises the step of administering to a subject a therapeutically effective amount of a peptide according to the invention or a composition according to the invention.

In preferred embodiments, the administering is carried out by a route selected from the group consisting of subcutaneous injection, intraperitoneal injection, intramuscular injection, bath immersion, and oral administration. More preferably, the administering is carried out by a route selected from the group consisting of subcutaneous injection, intramuscular injection and oral administration. Most preferably, the administering is carried out by subcutaneous injection.

Preferably, the tick infestation is selected from the group consisting of infestations by ticks of the genus *Ixodes,* and infestations by ticks of the genus *Dermacentor.* In a preferred embodiment, the infestation is by a tick from the genus *Ixodes,* and more preferably the infestation is by a tick from the species *Ixodes ricinus.* In a preferred embodiment of the present invention the infestation is by a tick from the genus *Dermacentor,* and more preferably the infestation is by a tick from the species *Dermacentor reticulatus.*

In preferred embodiments, the condition associated with a tick infestation in relation to the invention is selected from the group consisting of Lyme disease, borreliosis, tick-borne encephalitis, anaplasmosis, tularemia, rickettsiosis, Omsk hemorrhagic fever, and canine babesiosis.

In a preferred embodiment, the subject is selected from the group consisting of a dog, a cat, a rabbit, and a horse. In another preferred embodiment, the subject is selected from the group consisting of a dog, a cat, a rabbit, a horse, and other susceptible wild and domestic hosts. In a preferred embodiment the subject is selected from the group consisting of rabbits and dogs.

In a preferred embodiment the subject is a rabbit. In a preferred embodiment, the rabbit is selected from the genus *Oryctolagus* or the genera, *Pentalagus, Bunolagus, Nesolagus, Romerolagus, Brachylagus, Sylvilagus, Oryctolagus, Poelagus.* In a preferred embodiment, the dog is selected from the genus *Canis.* In a preferred embodiment, the rabbit is from the species *Oryctolagus cuniculus.* In a preferred embodiment, the dog is from the species *Canis lupus familiaris.* In a preferred embodiment, the cat is selected from the genus *Felis.* In a preferred embodiment, the cat is from the species *Felis catus,* also known as *Felis silvestris catus.*

One aspect of the present invention provides a method of controlling or the prophylactic treatment of a tick infestation and/or a condition associated with a tick infestation, in a subject, wherein the controlling or the prophylactic treatment is as defined herein in relation to the invention, and wherein the subject is as defined herein in relation to the invention.

Another aspect of the present invention provides a vector comprising a nucleic acid sequence encoding a peptide according to the invention. In one embodiment, the vector comprises at least two nucleic acid sequences encoding at least two different peptides according to the invention.

It will be understood that these nucleic acid sequences in relation to the invention may be, by way of techniques understood by one of skill in the art, cloned into vectors that enable expression of said nucleic acids in a host cell proceeded by isolation and purification of the peptides utilized as part of the composition of the invention.

In preferred embodiments, the vector comprises a nucleic acid sequence having a sequence identity of at least about 80%, 85%, 90% or 95% as compared to the nucleic acid sequences that are selected from the group consisting of SEQ ID 082, SEQ ID 216, SEQ ID 391, SEQ ID 749, SEQ ID S2, SEQ ID S8, SEQ ID S10, SEQ ID S12, SEQ ID S14, SEQ ID S16, SEQ ID S17, SEQ ID S18, SEQ ID S20. In a preferred embodiment the vector according to the invention comprises a nucleic acid sequence that is a heterologous sequence that is operably linked to one or more regulatory sequences as part of the vector of the invention.

Another aspect of the present invention provides an *in vitro* host cell comprising the vector of the present invention. In preferred embodiments, the *in vitro* host cell according to the invention is a prokaryotic cell. It will be understood that the *in vitro* host cell is alive.

Yet a further aspect of the invention is related to the use of a peptide according to the invention, or a composition according to the invention, in the preparation of a vaccine. In a preferred embodiment, said vaccine is a tick vaccine.

In some embodiments, the peptide of the invention having a sequence identity of at least 80% with an amino acid sequence selected from the group consisting of SEQ ID 082, SEQ ID 216, SEQ ID 391, SEQ ID 749, SEQ ID S2, SEQ ID S8, SEQ ID S10, SEQ ID S12, SEQ ID S14, SEQ ID S16, SEQ ID S17, SEQ ID S18, SEQ ID S20, may have undergone a conservative amino acid substitution in said amino acid sequence selected from said group. In a preferred embodiment, a conservative amino acid substitution will be any one that occurs within one of the following six groups:
1. Small aliphatic, substantially non-polar residues: Ala, Gly, Pro, Ser, and Thr;
2. Large aliphatic, non-polar residues: Ile, Leu, Val and Met;
3. Polar, negatively charged residues and their amides: Asp and Glu;
4. Amides of polar, negatively charged residues: Asn and Gin;
5. Polar, positively charged residues: Arg and Lys; His; and
6. Large aromatic residues: Trp, Tyr and Phe.

In a preferred embodiment, a conservative amino acid substitution will be any one of the following, which are listed as Native Residue (Conservative Substitutions) pairs: Ala (Ser); Arg (Lys); Asn (Gin; His); Asp (Glu); Gin (Asn); Glu (Asp); Gly (Pro); His (Asn;GIn); Ile (Leu; Val); Leu (Ile; Val); Lys (Arg; Gin; Glu); Met (Leu; Ile); Phe (Met; Leu; Tyr); Ser (Thr); Thr (Ser); Trp (Tyr); Tyr (Trp; Phe); and Val (Ile; Leu).

In a presently preferred embodiment the composition according to the invention is formulated for administration to dogs (preferably from the species *Canis lupus familiaris*) and/or cats (preferably from the species *Felis catus*). In further embodiments of the invention the vaccine is formulated for administration by one or more routes selected from the group consisting of subcutaneous injection, intramuscular injection and oral administration.

A "variant" peptide refers herein to a peptide, which differs in amino acid sequence from a "parent" vaccine peptide amino acid sequence by virtue of addition, deletion, and/or substitution of one or more amino acid residue(s) in the parent peptide sequence and retains at least one desired activity of the parent vaccine peptide. For example, the variant may comprise at least one, for example from about one to about ten, and preferably from about two to about five, substitutions in one or more amino acid sequences of the peptide to be used as part of the vaccine of the present invention. Ordinarily, the variant will have an amino acid sequence having at least 50% amino acid sequence identity with the parent amino acid sequences, preferably at least 65%, more preferably at least 70%, more preferably at least 75%, more preferably at least 80%, more preferably at least 85%, more preferably at least 90%, and most preferably at least 95% sequence identity. Identity or homology with respect to this sequence is defined herein as the percentage of amino acid residues in the candidate sequence that are identical with the parent peptide residues, after aligning the sequences and introducing gaps, if necessary, to achieve the maximum percent sequence identity. In general, none of N-terminal, C-terminal, or internal extensions, deletions, or insertions into the peptide sequence shall be construed as affecting sequence identity or homology. The variant retains the ability to elicit an immune response and preferably has desired activities, which are superior to those of the parent peptide. Variant peptides may be fully functional or may lack function in one or more activities. Moreover, polypeptides often contain amino acids other than the twenty-two "proteinogenic" amino acids. In preferred embodiments, the peptides of the invention consist of proteinogenic amino acids (*i*.*e*. alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, valine, selenocysteine, and pyrrolysine), preferably as L-enantiomers (apart from glycine that has no chiral carbon and therefore has no enantiomers). Further, many amino acids, including the terminal amino acids, may be modified by natural processes, such as processing and other post-translational modifications, or by chemical modification techniques well known in the art. Known modifications include, but are not limited to, acetylation, acylation, ADP-ribosylation, amidation, covalent attachmentof flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. Such modifications are well known to those of skill in the art and have been described in great detail in the scientific literature. Several particularly common modifications, glycosylation, lipid attachment, sulfation, gamma-carboxylation of glutamic acid residues, hydroxylation and ADP ribosylation. Accordingly, the peptides of the present invention also encompass derivatives or analogs in which a substituted amino acid residue is not one encoded by the genetic code. Similarly, the additions and substitutions in the amino acid sequence as well as variations, and modifications just described may be equally applicable to the amino acid sequence of peptide and/or epitope or peptides thereof, and are thus encompassed by the present invention.

A "therapeutically effective amount" or "immunogenically effective amount" refers to an amount of an active ingredient, for example a peptide or composition according to the invention, sufficient to effect beneficial or desired results when administered to a subject. An effective amount can be administered in one or more administrations, applications or dosages. A therapeutically effective amount of a peptide or composition according to the invention may be readily determined by one of ordinary skill in the art. An effective amount can be administered in one or more administrations. For purposes of this invention, an effective amount of peptide or composition according to the invention is an amount sufficient to induce a protective immune response in the subject that will control tick infestations. The therapeutically effective amount will vary depending upon the particular subject and condition being treated, the weight and age of the subject, the severity of the disease condition, the particular compound chosen, the dosing regimen to be followed, timing of administration, the manner of administration and the like, all of which can readily be determined by one of ordinary skill in the art.

As used herein, "treatment" is an approach for obtaining beneficial or desired clinical results. For purposes of this invention, beneficial or desired clinical results include, but are not limited to preventing and/or controlling tick infestations in animals.

A "variant" nucleic acid, refers herein to a molecule which differs in sequence from a -"parent" nucleic acid. Polynucleotide sequence divergence may result from mutational changes such as deletions, substitutions, or additions of one or more nucleotides. Each of these changes may occur alone or in combination, one or more times in a given sequence.

As used herein, "vector" means a construct, which is capable of delivering, and preferably expressing, one or more gene(s) or sequence(s) of interest in a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. Vectors, as described herein, have expression control sequences meaning that a nucleic acid sequence that directs transcription of a nucleic acid. An expression control sequence can be a promoter, such as a constitutive or an inducible promoter, or an enhancer. The expression control sequence is 'operably linked' to the nucleic acid sequence to be transcribed. A nucleic acid is "operably linked" when it is placed into a functional relationship with another nucleic acid sequence. For example, DNA for a pre-Sequence or secretory leader is operably linked to DNA for a polypeptide if it is expressed as a pre-protein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

Before the present methods are described, it is to be understood that this invention is not limited to particular methods, and experimental conditions described, as such methods and conditions may vary. It is also to be understood that the terminology used herein is for the purpose of describing particular embodiments only, and is not intended to be limiting. Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described.

The present invention is further illustrated and supported by the following examples. However, these examples should in no way be considered to further limit the scope of the invention. To the contrary, one having ordinary skill in the art would readily understand that there are other embodiments, modifications, and equivalents of the present invention without departing from the spirit of the present invention and/or the scope of the appended claims.

It should be understood that this invention is not limited to the particular methodology, protocols, and reagents described herein and as such may vary. The terminology used herein is for the purpose of describing particular embodiments only, and is not intended to limit the scope of the present invention. Unless otherwise defined, scientific and technical terms used in connection with the invention described herein shall have the meanings that are commonly understood by those of ordinary skill in the art. Further, unless otherwise required by context, singular terms shall include pluralities and plural terms shall include the singular. Generally, nomenclatures utilized in connection with, and techniques of cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described, but not limited to the various general and more specific references that are cited and discussed throughout the present specification (see for example Sambrook et al. MOLECULAR CLONING: LAB. MANUAL, 3rd ed., Cold Spring Harbor Lab. Press, Cold Spring Harbor, N.Y., 2001; Ausubel et al. Current Protocols in Molecular Biology, New York: Greene Publishing Association JWiley Interscience, Oligonucleotide Synthesis (M. J. Gait, ed.,1984); Methods in Molecular Biology, Humana Press; Cell Biology: A Laboratory Notebook (J. E. Cellis, ed., 1998); Introduction to Cell and Tissue Culture (J.M. Mather and P. E. Roberts, 1998), Plenum Press; Cell and Tissue Culture: Laboratory Procedures (A. Doyle, J. B. Griffiths, and D. G. Newell, eds., 1993-1998), J. Wiley and Sons; Methods in Enzymology (Academic Press, Inc.); Handbook of Experimental Immunology (D. M. Weir and C. C. Blackwell, eds.); Gene Transfer Vectors for Mammalian Cells (J. M. Miller and M. P. Calos, eds., 1987); Current Protocols in Molecular Biology (F. M. Ausubelet al., eds., 1987); PCR: The Polymerase Chain Reaction, (Mullis et al., eds., 1994); Current Protocols in Immunology (E. Coligan et al., eds., 1991); Short Protocols in Molecular Biology (Wiley and Sons, 1999); immunobiology (C. A. Janeway and P. Travers, 1997); Antibodies (P. Finch, 1997); Antibodies: a practical approach (D. Catty., ed., IRL Press, 1988-1989); Monoclonal antibodies: a practical approach (P. Shepherd and C. Dean, eds., Oxford University Press, 2000); Using antibodies: a laboratory manual (E. Harlow and D. Lane (Cold Spring Harbor Laboratory Press, 1999); The Antibodies (M. Zanetti and J. D. Capra, eds., Harwood Academic Publishers, 1995).

Other than in the operating examples, or where otherwise indicated, all numbers expressing quantities of ingredients or reaction conditions used herein should be understood as modified in all instances by the term "about". All publications identified are expressly incorporated herein by reference for the purpose of describing and disclosing, for example, the methodologies described in such publications that might be used in connection with the present invention. These publications are provided solely for their disclosure prior to the filing date of the present application. Unless otherwise expressly defined herein, terms of art used in this specification will have their art-recognized meanings attributed to them in international patent law.

### EXAMPLES

### EXAMPLE 1: Identification of candidate tick protective peptides.

A reverse vaccinology approach based on transcriptomics and proteomics data was used for the discovery of tick protective peptides for the control of *I. ricinus* and *D. reticulatus* infestations, particularly in dogs. Tick larvae and adult salivary glands were selected for analysis. Tick larvae are the first developmental stage to infest hosts and acquire infection or transmit pathogens that are transovarially transmitted. Salivary glands produce a cocktail of anti-clotting, anti-platelet, anti-inflammatory, vasodilatory and immunomodulatory proteins that interfere with host defenses to aid in tick blood feeding and pathogen infection and transmission.

Tick *I. ricinus* unfed larvae whole internal tissues (IL) and adult salivary glands (ISG), and *D. reticulatus* unfed larvae whole internal tissues (DL) and adult salivary glands (DSG) were processed. RNA and proteins were extracted from approximately 500 larvae using the AllPrep DNA/RNA/Protein Mini Kit (Qiagen, Valencia, CA, USA) and from 50 salivary glands (25 females and 25 males) using Tri Reagent (Sigma-Aldrich, St. Louis, MO, USA) according to manufacturer instructions. RNA was purified with the RNeasy MinElute Cleanup Kit (Qiagen, Valencia, CA, USA) and characterized using the Agilent 2100 Bioanalyzer (Santa Clara, CA, USA) in order to evaluate the quality and integrity of RNA preparations. RNA concentration was determined using the Nanodrop ND-1000 (NanoDrop Technologies Wilmington, Delaware USA). Protein concentration was determined using the Pierce BCA Protein Assay Kit (Thermo Scientific, Rockford, IL, USA) with BSA as standard.

Purified RNAs were used for library preparation using the TruSeq RNA sample preparation kit v.1 and the standard low throughput procedure (Illumina, San Diego, CA, USA). One µg total RNA was used as starting material for library preparation with the exception of the DL sample for which 0.7 µg were used. Messenger RNA was captured using poly-dT magnetic beads and purified polyA+ RNA was chemically fragmented and reverse-transcribed. Remaining RNA was enzymatically removed and the second strand generated following an end repair procedure and preparation of double-stranded cDNA for adaptor ligation. Adaptor oligonucleotides containing the signals for subsequent amplification and sequencing were ligated to both ends and cDNA samples were washed using AMPure SPRI-based magnetic beads (Beckman Coulter, IZASA, Barcelona, Spain). Adapters contained identifiers, which allow multiplexing in the sequencing run. An enrichment procedure based on PCR was then performed to ensure that all molecules in the library conserved the adapters at both ends. The number of PCR cycles was adjusted to 10 for all samples except for DL which needed up to 15 cycles. The final amplified library was checked again on a BioAnalyzer 2100 (Agilent, Santa Clara, CA, USA). Libraries were titrated by quantitative PCR using a reference standard to characterize molecule concentration per library (IL, 5.51 nM; ISG, 62.16 nM; DL12.44 nM; DSG 100.95 nM). Libraries were denatured and seeded on the respective lanes of the flowcells at a final concentration after re-naturalization of 10-14 pM. IL and ISG samples were run in single-nucleotide flowcells while DL and DSG samples were run in double-nucleotide flowcells to allow for pair-end sequencing. After binding, clusters were formed in the flowcells by local amplification using a Cluster Station apparatus (Illumina). Following sequencing primer annealing, flowcells were loaded into a GAIIx equipment (Illumina) to perform sequencing using the TruSeq® system (Illumina). IL and ISG samples were run under a 1x75 bp single-end read protocol while DL and DSG samples were run under a pair-end 2x100 bp protocol for *de novo* sequencing. After sequencing and quality filtering, reads were split according to their different identifiers and fastq files were generated to proceed to quality analysis and gene expression analysis and/or *de novo* transcript assembly.

The reads from both IL and ISG samples were mapped to the reference *I. scapularis* genome (assembly JCVI_ISG_i3_1.0; http://www.ncbi.nlm.nih.gov/nuccore/NZ_ABJB000000000) using a TopHat-Cufflinks-Cuffdiff pipeline. Transcript abundance was estimated using Cufflinks by analyzing the number of reads mapped to each transcript and handling the deviations due to the sample preparation. Finally, differential expression between IL and ISG was characterized using Cuffdiff, integrated into the Cufflinks package. For analysis of genes coding for secreted proteins, 6 subcellular localization predictors were chosen: WoLF PSORT (http://wolfpsort.org), TargetP (http://www.cbs.dtu.dk/services/TargetP/), Protein Prowler (http://pprowler.itee.uq.edu.au/pprowler_webapp_1-2/), SLP-Local (http://sunflower.kuicr.kyoto-u.ac.jp/∼smatsuda/slplocal.html), PredSL (http://hannibal.biol.uoa.gr/PredSL/) and CELLO (http://cello.life.nctu.edu.tw). All these tools have web services that enable batch protein sequence submissions. For *D. reticulatus* transcriptomics data, the 21,838,507 reads (∼2.2Gb) in DSG were compared against the selected *I*. *ricinus* reference gene set using PROmer. The query sequences were considered in their 3 reading frames and the reference sequences only in the frame 1. PROmer results were filtered by selecting the hits with a minimum length of 60 bp for the extended similarity region and a minimum identity of 80% in this region. Reads with hits fulfilling these requirements were clustered to the selected gene with which they have PROmer hits. The reads belonging to each cluster were then assembled with Velvet to produce a set of contigs clustered to the reference genes. A BLASTX was then done against all proteins from Ixodidae included in the nr database. For each cluster of contigs, we chose the most similar protein to which the contigs aligned with more similarity, and substituted that protein as the new reference protein for that cluster. Differential gene expression between DL and DSG samples was determined using X² test statistics with Bonferroni correction (P = 0.05) in the IDEG6 software (http://telethon.bio.unipd.it/bioinfo/IDEG6_form/).

Proteins were resuspended in 5% SDS and frozen at -20°C until use. Two hundred µg of protein extracts from the four samples, IL, ISG, DL and DSG, were mixed with the same quantity of Laemmli sample buffer x2 and applied using a 5-well comb on a conventional SDS-PAGE gel (1.5 mm-thick, 4% stacking, 10% resolving). The electrophoretic run was stopped as soon as the front entered 3 mm into the resolving gel, so that the whole proteome became concentrated in the stacking/resolving gel interface. The unseparated protein bands were visualized by Coomassie Brilliant Blue R-250 staining, excised, cut into cubes (2 x 2 mm) and treated with 0.1% RapiGest SF surfactant (Waters, Milford. MA, USA) according to manufacturer's protocol in order to enhance the subsequent in-gel trypsin digestion. After RapiGest treatment, samples were digested overnight at 37 °C with 60 ng/µl trypsin (Promega, Madison, WI, USA) at 5:1 protein:trypsin (w/w) ratio in 50 mM ammonium bicarbonate, pH 8.8 containing 10% (v/v) acetonitrile. The resulting tryptic peptides from each proteome were extracted by 1 h-incubation in 12 mM ammonium bicarbonate, pH 8.8. Trifluoroacetic acid (TFA) was added to a final concentration of 1% and the peptides were finally desalted onto C18 OASIS HLB extraction cartridges (Waters, Milford, Massachusetts, USA) and dried-down prior to reverse phase high performance liquid chromatography (RP-HPLC) method coupled with mass spectrometry (RP-HPLC-MS/MS) analysis using a Surveyor LC system coupled to an ion trap mass spectrometer model LCQ Fleet (Thermo-Finnigan, San Jose, CA, USA). The LCQ Fleet was programmed to perform a data-dependent MS/MS scan on the 3 most intense precursors detected in a full scan from 400 to 1600 amu (1 µscan, 200 ms injection time). Protein identification was conducted using SEQUEST algorithm (Proteome Discoverer 1.1 package, Thermo Finnigan), allowing optional modifications in Methionine (oxidation) and Cysteine (carboxamidomethylation).

The MS/MS raw files were searched against the Ixodidae Uniprot database (28,771 entries in February 2012) with the following constraints: tryptic cleavage after Arginine and Lysine, up to two missed cleavage sites, and tolerances of 0.5 Da for precursor ions and 0.8 Da for MS/MS fragment ions. Protein assignations were first filtered with a SEQUEST XCorr > 2, but only proteins with false discovery rate (FDR) ≤ 0.01 and identified with at least 4 peptides were selected for further analysis from the Ixodidae database. The MS/MS data was also used to search against the database of predicted secreted proteins encoded by the selected top 1,000 expressed genes in both IL and ISG. In this case, proteins with sequence span > 2% were selected for further analysis. Differential protein representation between L and SG samples was determined using X² test statistics with Bonferroni correction (P = 0.001) in the IDEG6 software (http://telethon.bio.unipd.it/bioinfo/IDEG6_form/).

Gene ontology (GO) analysis for Molecular Function (MF), Cellular Component (CC) and/or Biological Process (BP) was conducted using the cDNA Annotation System software (dCAS; Bioinformatics and Scientific IT Program (BSIP), Office of Technology Information Systems (OTIS), National Institute of Allergy and Infectious Diseases (NIAID), Bethesda, MD, USA; http://exon.niaid.nih.gov), AmiGO (http://amigo.geneontology.org/cgibin/amigo/search.cgi) and UniProt (http://www.uniprot.org) using nonredundant sequence database (nr) and databases of tick-specific sequences (http://www.ncbi.nlm.nih.gov and http://www.vectorbase.org/index.php).

### EXAMPLE 2: Isolation, production and vaccine formulation of selected tick protective peptides.

An algorithm was developed using a reverse vaccinology approach to tick protective peptide discovery. The algorithm included the generation and use of transcriptomics and proteomics data in tick L and SG and selection of gene transcripts and proteins highly represented in both samples and encoding for functionally important secreted proteins. The initial dataset was obtained from *I. ricinus* because of the availability of the *I. scapularis* genome sequence for data mining (Gulia-Nuss et al., 2017. Nature Commun. 7:10507). Genes selected in *I*. *ricinus* as encoding for tick candidate protective peptides were then used to find orthologous genes in *D. reticulatus.* Tick L and SG were selected to cover both initial (larvae) and final (adult) developmental stages and tissues that are essential for pathogen acquisition and transmission, respectively.

Using the trascriptomics data that was obtained from IL and ISG samples, the top 1,000 most expressed genes in both samples and genes encoding for secreted proteins were selected. GO analysis for BP was then used to select for functionally relevant genes. The Proteomics data was used for mining databases of Ixodidae and predicted secreted proteins. Selected genes (N=36) expressed or overexpressed in IL and ISG were finally subjected to GO analysis for BP, MF and CC to remove two genes encoding for nuclear DNA-binding proteins, resulting in a final set of 34 selected genes encoding for candidate protective peptides. *D. reticulatus* data were compared against the selected *I. ricinus* reference gene set, and resulted in the identification of 25 candidate protective peptides.

All *I. ricinus* genes encoding for candidate tick protective peptides were identified using the *I. scapualris* reference database and therefore the sequences corresponding to *I. ricinus* were first amplified by RT-PCR using primers designed for *I. scapualris* sequences together with selected *D. reticulatus* orthologs. The genes that did not produce an amplicon after RT-PCR were eliminated for further analysis, resulting in 17 and 21 genes encoding for candidate protective peptides selected in *I. ricinus* and *D. reticulatus,* respectively for the production of recombinant proteins and vaccination studies.

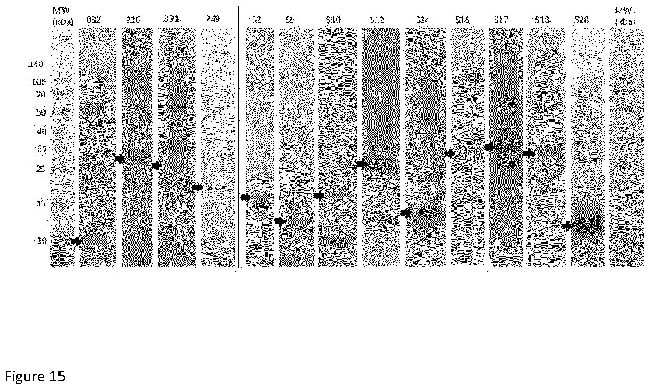

For gene expression and the production of the recombinant proteins, *E. coli* BL21 Star (DE3) One Shot cells (Invitrogen-Life Technolgies, Inc., Grand Island, NY, USA) were transformed with the target gene cloned into the pET101/D-TOPO expression vector (Invitrogen-Life Technolgies). Recombinant *E. coli* were inoculated into 10 ml of Luria-Bertani (LB) containing 50 µg/ml ampicillin (Sigma-Aldrich, St Louis, MO, USA) and 0.4% glucose (Laboratories CONDA S.A., Madrid, Spain) and kept growing overnight at 37 °C with shaking. Two ml of the overnight culture was propagated into 250 ml flasks containing 50 ml LB, 50 µg/ml ampicillin and 0.4% glucose for 2 h at 37 °C and 200 rpm, and then for 4 h after addition of 0.5 mM final concentration of isopropyl-B-d-thiogalactopyranoside (IPTG, Sigma-Aldrich) for induction of gene expression. The cells were harvested by centrifugation at 3,900 x g for 15 min at 4 °C and stored at -80 °C for protein purification. One g of the cells harvested after induction of gene expression were resuspended in 5 ml of lysis buffer (100 mM Tris-HCl, pH 7.5, 250 mM NaCl, 7 M Urea, 10 mM imidazole) containing protease inhibitor (Ref. 04693132001, Roche, San Cugat del Vallés, Barcelona, Spain) and disrupted using a cell sonicator (Model MS73; Bandelin Sonopuls, Berlin, Germany). After disruption, the insoluble protein fraction containing the recombinant peptides as inclusion bodies were collected by centrifugation at 15,000 x g for 15 min at 4 °C and stored at -20 °C before purification. The purification of the recombinant protein was conducted using the automated Maxwell 16 Polyhistidine Protein Purification Kit (Promega, Madison, WI, USA). The eluted fraction containing the purified denatured proteins was dialyzed against 1000 volumes of PBS (137 mM NaCl, 2.7 mM KCl, 10 mM NazHPO₄, 1.8 mM KH₂PO₄), pH 7.4 for 12 h at 4 °C. Recombinant proteins were then concentrated using an Amicon Ultra-15 ultrafiltration device (cut off 10 kDa) (Millipore-Merck, Darmstadt, Germany), and adjusted to 0.5 mg/ml. Protein concentration was determined using bicinchoninic acid (Pierce BCA Protein Assay Kit, Thermo Scientific, Rockford, IL, USA). Exemplary results of recombinant peptides can be seen in Figure 15.

For vaccine formulation, recombinant proteins or saline control were adjuvated in Montanide ISA 50 V2 (Seppic, Paris, France) for rabbit trials or Montanide PET GEL A (Seppic), a ready-to-disperse polymeric adjuvant designed to improve the safety and efficacy of vaccines for companion animals for dog trials to a final protein concentration of 250 µg/ml.

### EXAMPLE 3: Vaccine efficacy of tick protective peptides in rabbits.

The 17 and 21 genes encoding for candidate protective peptides identified in *I. ricinus* and *D. reticulatus,* respectively were used for the production of recombinant proteins and initial vaccination screening in rabbits.

Three two-year-old white rabbits (*Oryctolagus cuniculus*) per group were injected subcutaneously (dorsum between shoulders) at days 0 (T1) and 14 (T2) with 0.2 ml (50 µg) doses of recombinant protein vaccine formulations or adjuvant/saline using a syringe with removable needle (0.45 x 13 mm). Two weeks after the last immunization (day 28; T3), rabbits in vaccinated and control groups were infested with 200 tick larvae of approximately 21 days after hatching placed on bags located on each rabbit's shaved ear. Immunizations, tick larval infestations, collections and evaluations were done blinded and the key to the experimental groups was not disclosed until the end of the experiment. Engorged tick larvae were collected, counted and weighted as they dropped off between days 31 to 34. Fed larvae were incubated at 21 °C, 80-82 % humidity with 7 h dark and 17 h light photoperiod until molting. The tick larvae successfully molting to nymphal stage were collected and counted between days 51 to 65. The number of engorged larvae, weight/larvae, and percent of larvae molting to nymphs were evaluated. Data were analyzed statistically to compare results for each tick species between individuals fed on vaccinated and adjuvant/saline injected control rabbits by Student's t-test with unequal variance (P = 0.05). Vaccine efficacy (E) was calculated as E (%) = 100 [1-(DL x DM_{N})], where DL is the reduction in the number of engorged larvae and DM_{N} is the reduction in the percent of larvae molting to nymphs in ticks fed on vaccinated rabbits when compared to the controls fed on adjuvant/saline injected rabbits. Only parameters with statistically significant differences were included in E calculation.

The tick protective peptides with vaccine efficacy (E) higher than 40% in vaccinated rabbits, resulted in the peptides described in Table 1 with protective capacity against tick infestations in rabbits.

**TABLE 1. Results of the vaccination trial in rabbits, and tick protective peptides selected for vaccination in dogs.**

| **SEQ ID** | **Vaccine efficacy (E)** |
|---|---|
| 082 | 83% |
| 216 | 85% |
| 391 | 86% |
| 749 | 95% |
| S2 | 55% |
| S8 | 59% |
| S10 | 44% |
| S12 | 68% |
| S14 | 52% |
| S16 | 75% |
| S17 | 65% |
| S18 | 61% |
| S20 | 93% |

### EXAMPLE 4: Vaccine efficacy of tick protective peptides in dogs.

The criterion for the final selection of tick protective peptides for vaccination trials in dogs was based on a vaccine efficacy (E) higher than 40% in vaccinated rabbits, resulting in the peptides described in Table 1.

Three randomly mixed male and female dogs (age > 6 months; body weight > 2 Kg) per group were assigned to *I. scapularis* or *D. reticulatus* trial and injected subcutaneously (loose skin on the side of the chest) at days 0 (T1) and 21 (T2) with 0.2 ml (50 µg) doses of recombinant protein vaccine formulations or adjuvant/saline using a syringe with removable needle (0.45 x 13 mm). Two weeks after the last immunization (day 36; T3), dogs in vaccinated and control groups were infested with *I. scapularis* or *D. reticulatus* 25:30 (female:male ratio) adults of at least 10 days after molting and 100 nymphs of approximately 15 days after hatching placed on feeding chambers (one chamber per tick stage) glued on each dog's shaved flank. Immunizations, tick larval infestations, collections and evaluations were done blinded and the key to the experimental groups was not disclosed until the end of the experiment. Engorged tick females were collected, counted and weighted as they dropped off between days 41 and 48. Fed females were incubated at 21 °C, 80-82 % humidity with 24 h dark photoperiod for approximately one month until oviposition. Egg mass per tick was weighted and incubated for fertility determined by counting the number of larvae per tick. Engorged tick nymphs were collected and counted as they dropped off between days 40 to 43. Detached engorged nymphs were incubated at 21 °C, 80-82 % humidity with 8 h dark and 16 h light photoperiod until molting. The tick nymphs successfully molting to adults were collected and counted approximately one month after collection. The number of engorged nymphs and adult females, weight/female, oviposition (egg mass/tick), fertility (No. hatching larvae), and number of nymphs molting to adults were evaluated. Data were analyzed statistically to compare results for each tick species between individuals fed on vaccinated and adjuvant/saline injected control rabbits by Chi²-test (P = 0.05). Vaccine efficacy (E) was calculated as E (%) = 100 [1-(DN x DM_{A} x DT x DO x DF)], where DN is the reduction in the number of engorged nymphs, DM_{A} is the reduction in the number of nymphs molting to adults, DT is the reduction in the number of engorged female ticks, DO is the reduction in oviposition, and DF is the reduction in fertility in ticks fed on vaccinated rabbits when compared to the controls fed on adjuvant/saline injected dogs. Only parameters with statistically significant differences were included in E calculation as demonstrated in Table 2.

**TABLE 2. Results of the vaccination trial in dogs.**

| **SEQ ID** | **DN (%)** | **DM_{A} (%)** | **DT (%)** | **DW_{A} (%)** | **DO (%)** | **DF (%)** | **E (%)** |
|---|---|---|---|---|---|---|---|
| 082 | 6 | 0 | 0 | 0 | 0 | 0 | 6 |
| 216 | 20 | 5 | 33 | 5 | 0 | 67 | 83 |
| 391 | 37 | 33 | 9 | 0 | 49 | 68 | 94 |
| 749 | 0 | 0 | 0 | 0 | 0 | 0 | 0 |
| S2 | 40 | 41 | 0 | 22 | 69 | 0 | 89 |
| S8 | 64 | 60 | 0 | 0 | 63 | 0 | 95 |
| S10 | 10 | 9 | 0 | 1 | 71 | 0 | 76 |
| S12 | 11 | 12 | 0 | 0 | 65 | 9 | 75 |
| S14 | 0 | 0 | 0 | 0 | 75 | 0 | 75 |
| S16 | 0 | 0 | 0 | 0 | 60 | 0 | 60 |
| S17 | 41 | 50 | 11 | 0 | 43 | 0 | 85 |
| S18 | 55 | 60 | 0 | 0 | 43 | 4 | 90 |
| S20 | 55 | 63 | 0 | 6 | 67 | 14 | 95 |

Vaccination with the vaccine comprising the formulation as described above with peptides with SEQ IDs 216, 391, S2, S8, S10, S12, S14, S16, S17, S18 and S20 reduced tick infestations by affecting various developmental stages with an overall vaccine efficacy (E) higher than 60%.

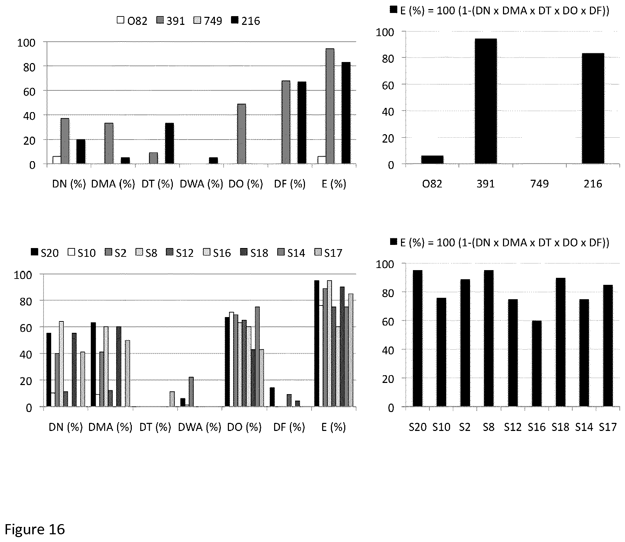

Graphic representation of the data in Table 2 can be seen in Figure 16, graphically depicting the results of vaccination with tick peptides in reducing *I. ricinus* and *D. reticulatus* infestations in dogs.

### EXAMPLE 5: Immunogenicity of tick protective peptides in rabbits and dogs.

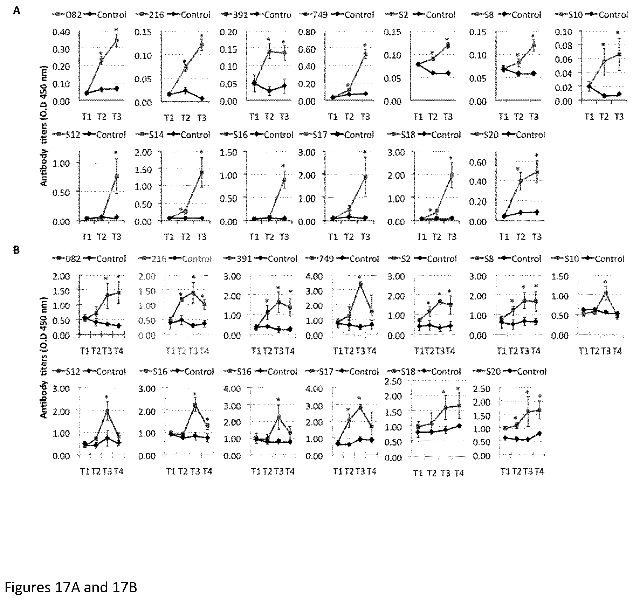

Recombinant peptides were produced and formulated, and rabbits or dogs vaccinated as described in Examples 2,3 and 4. In rabbits, blood samples were collected from each rabbit before each immunization (T1 and T2) and tick infestation (T3) into sterile tubes and maintained at 4 °C until arrival at the laboratory. In dogs, blood samples were collected from each dog before each immunization (T1 and T2), tick infestation (T3) and at day 75 (T4) into sterile tubes and maintained at 4 °C until arrival at the laboratory. Serum was then separated by centrifugation and stored at -20 °C. An indirect ELISA test was performed to detect IgG antibodies against recombinant peptides in serum samples from vaccinated and control rabbits collected at T1-T3. High absorption capacity polystyrene microtiter plates were coated with 50 µl (0.02 µg/ml solution of purified recombinant proteins) per well in carbonate-bicarbonate buffer (Sigma-Aldrich). After an overnight incubation at 4 °C, coated plates were blocked with 200 µl/well of blocking solution (5% skim milk in PBS). Serum samples or PBS as negative control were diluted (1:10, v/v) in blocking solution and 50 µl/well were added into duplicate wells of the peptide-coated plates. After an overnight incubation at 4 °C, the plates were washed three times with a washing solution (PBS containing 0.05% Tween 20). A goat anti-rabbit IgG-peroxidase conjugate (Sigma-Aldrich) was added (diluted 1:500 in blocking solution) and incubated at RT for 1 h. After three washes with washing solution, 200 µl/well of substrate solution (Fast OPD, Sigma-Aldrich) was added. Finally, the reaction was stopped with 50 µl/well of 3N H₂SO₄ and the optical density (OD) was measured in a spectrophotometer at 450 nm. Antibody titers in vaccinated and control rabbits were expressed as the OD₄₅₀ₙₘ (OD_{rabbit sera} - OD_{PBS control}) and compared between vaccinated and control groups by ANOVA test (P = 0.05). All peptides showed an peptide-specific IgG antibody response in vaccinated rabbits. Exemplary results of vaccine peptide immunogenicity in rabbits can bee seen in Figure 17A. Exemplary results of vaccine peptide immunogenicity in dogs can bee seen in Figure 17B.

## Claims

1. An isolated peptide identified in the tick species *Ixodes ricinus* and/or *Dermacentor reticulatus,* wherein said peptide is identified in both a larva of said tick species, and a salivary gland of an adult tick of the same tick species as said larva, wherein said peptide comprises an amino acid subsequence of SEQ ID 1, wherein said peptide consists of a number of amino acid residues in a range of from 10 to 350, preferably in a range of from 20 to 300.

2. A peptide according to claim 1, having a sequence identity of at least 80%, preferably at least 85%, with an amino acid sequence selected from the group consisting of SEQ ID 082, SEQ ID 216, SEQ ID 391, SEQ ID 749, SEQ ID S2, SEQ ID S8, SEQ ID S10, SEQ ID S12, SEQ ID S14, SEQ ID S16, SEQ ID S17, SEQ ID S18, SEQ ID S20, and variants or immunogenic fragments thereof.

3. A peptide according to claim 1 or 2, wherein the peptide is a recombinant peptide.

4. A peptide according to claim 1 or 2, wherein the peptide is a secreted peptide.

5. A method for identifying a protective peptide, comprising the steps of:
a) providing at least one tick larva, and a salivary gland of an adult tick, wherein the tick is *Ixodes ricinus* and/or *Dermacentor reticulatus*;
b) identifying at least one peptide that is expressed in both the tick larva, and the salivary gland of the adult tick.

6. An isolated peptide obtainable by the method of claim 5, preferably as defined in any one of claims 1 to 4.

7. A composition comprising an immunologically effective amount of at least one peptide according to any one of claims 1 to 4 or 6, preferably an immunologically effective amount of at least two different peptides according to any one of claims 1 to 4 or 6, said composition optionally comprising a pharmaceutically acceptable carrier.

8. A composition according to claim 7, comprising a pharmaceutically acceptable adjuvant, preferably selected from the group consisting of muramyl dipeptides, lipopolysaccharides, glucans, glycans, an oil-in-water emulsion, a water-in-oil emulsion, Freund's adjuvant, and acrylic acid polymers.

9. A composition according to claim 8, wherein the adjuvant is a water-in-oil emulsion.

10. A peptide as defined in any one of claims 1 to 4, or 6, for use as a medicament.

11. A peptide as defined in any one of claims 1 to 4, or 6, for use in the control or prophylactic treatment of a tick infestation and/or a condition associated with a tick infestation, in a subject, said treatment preferably comprising administering to a subject a therapeutically effective amount of the peptide by a route selected from the group of subcutaneous injection, intraperitoneal injection, intramuscular injection, bath immersion, and oral administration.

12. A peptide or composition for use according to claim 11. wherein the subject is selected from the group consisting of a dog, a cat, a rabbit, and a horse.

13. A vector comprising a nucleic acid sequence encoding the peptide according to any one of claims 1 to 4, or 6.

14. An *in vitro* host cell comprising the vector of claim 13.

15. Use of a peptide according to any one of claims 1 to 4, or 6, or a composition according to any one of claims 7 to 9, in the preparation of a tick vaccine.

16. A method of controlling or the prophylactic treatment of a tick infestation and/or a condition associated with a tick infestation, in a subject, wherein the controlling or the prophylactic treatment is as defined in claim 11 or 12.
